**Europäisches Patentamt**

**European Patent Office**

**Office européen des brevets**

(19)

(11) Veröffentlichungsnummer **0 040 416 B1**

(12)

# EUROPÄISCHE PATENTSCHRIFT

(45) Veröffentlichungstag der Patentschrift:
08.08.84

(21) Anmeldenummer: 81103761.3

(22) Anmeldetag: 15.05.81

(51) Int. Cl.³: **A 61 B 5/14, G 05 D 23/24**

(54) **Beheizter Messwertaufnehmer für physiologische Messgrössen mit einem eingebauten temperaturabhängig steuerbaren Schalter.**

(30) Priorität: 16.05.80 DE 3018863

(43) Veröffentlichungstag der Anmeldung:
25.11.81 Patentblatt 81/47

(45) Bekanntmachung des Hinweises auf die Patenterteilung:
08.08.84 Patentblatt 84/32

(84) Benannte Vertragsstaaten:
CH DE FR GB LI NL

(56) Entgegenhaltungen:
DE - A - 2 514 478
DE - A - 2 836 883
FR - A - 2 440 027
GB - A - 2 050 615
US - A - 3 500 074
US - A - 3 831 003

HOW THINGS WORK 1, 1979 Granada Publishing St.
ALBANS (GB) Seiten 250,251: Thermal Electric
Domestic Appliances.
MEDICAL AND BIOLOGICAL ENGINEERING, Band 13,
Nr. 3, Mai 1975 STEVENAGE (GB) P. EBERHARD et al.:
"Continuous pO2 monitoring in the neonate by skin
electrodes", Seiten 436 bis 442

(73) Patentinhaber: **Hellige GmbH,
Postfach 728 Heinrich-von-Stephan-Strasse 4,
D-7800 Freiburg (DE)**

(72) Erfinder: **Leist, Helmut, Dipl.-Ing., Waldallee 36,
D-7800 Freiburg/Lehen (DE)**

(74) Vertreter: **Patentanwälte TER MEER - MÜLLER -
STEINMEISTER, Triftstrasse 4, D-8000 München 22 (DE)**

## Beschreibung

Die Erfindung betrifft einen Meßwertaufnehmer für physiologische Meßgrößen entsprechend dem Oberbegriff des Patentanspruchs 1.

Zur Messung und/oder Registrierung des zeitlichen Verlaufs physiologischer Größen werden in der medizinischen Diagnostik in zunehmendem Maße Meßwertaufnehmer eingesetzt, die an einer Stelle des Körpers des Patienten auf der Haut befestigt werden. Zum Zwecke der Konstanthaltung der Temperatur des Aufnehmers und/oder der von ihm bedeckten Zone des Patientenkörpers wird der Meßwertaufnehmer selbst und/oder diese Körperzone erwärmt um eine Hyperämisierung zu erreichen. Bei Meßwertaufnehmern der hier in Rede stehenden Art ist in erster Linie an Sensoren gedacht, die zur Messung und Registrierung physiologischer Größen dienen, zum Beispiel Sensoren für die unblutige transkutane Messung und Registrierung physiologischer Größen, Sensoren für die Durchblutungsmessung von Körperorganen, Sensoren für die oximetrische Messung des Sauerstoffpartialdrucks ($pO_2$) im Blut, polarographische Sensoren für die Bestimmung der Konzentration von Gasen wie Sauerstoff und Kohlendioxyd im Blut, Sensoren für die pH-Wertbestimmung des Blutes sowie Sensoren für die Erfassung und Bestimmung von sonstigen Bestandteilen im Blut, z. B. von Alkohol oder Zucker.

Um insbesondere bei der transkutanen Anwendung von Meßwertaufnehmern in der medizinischen Diagnostik den Patienten vor einer Verbrennung oder Hautschädigung durch Übertemperatur zu schützen, die durch einen Defekt im Regelkreis für die Heizung oder auch durch grobe Bedienungsfehler entstehen kann, sind bisher relativ komplizierte und teuere Vorkehrungen im elektronischen Betriebsgerät für den Aufnehmer getroffen worden. Dabei ging man vor allem von der Auffassung aus, daß der Einbau eines Temperaturfühlers in den Meßwertaufnehmer ausreicht, um eine Regelung der dem Aufnehmer zugeführten Heizleistung zu erreichen. Es wurde auch schon daran gedacht, einen zweiten Temperaturfühler in den Meßwertaufnehmer einzubauen, um der Möglichkeit vorzubeugen, daß durch Ausfall des einzigen Temperaturfühlers die Temperatur im Aufnehmer zu hoch wird. Ein solcher redundanter Temperaturfühler erfordert jedoch zusätzliche Signalleitungen in der Zuleitung zum Meßwertaufnehmer, die erfahrungsgemäß selbst wieder das störanfälligste Glied der Meßkette sowie einen zusätzlichen elektronischen Aufwand darstellen. Dabei muß berücksichtigt werden, daß in der Zuleitung eine Vielzahl von Signalleitungen zusammengefaßt wird, die Zuleitung aber gleichwohl möglichst dünn und flexibel gehalten werden muß.

Aus der DE-A 2 514 478 ist eine Vorrichtung und ein Verfahren zur Messung des Partialdrucks von Gasen oder Dämpfen im Blut bekannt, wobei das messend zu erfassende Gas aus einer Gassammelkammer über eine Rohrleitung einem Massenspektrometer oder einem anderen, geeigneten Analyseinstrument zugeleitet wird. Ein Thermistor ist an einen Steuerschaltkreis zur Thermostatisierung der Gassammelkammer angeschlossen, über einen weiteren Thermistor wird die Hauttemperatur an der Meßstelle des Meßobjekts erfaßt. In der genannten Druckschrift ist jedoch weder eine zusätzliche Überwachung noch ein Eingreifen für den Fall beschrieben, daß ein Defekt am ersten Thermistor, am Steuerschaltkreis, an der Heizungseinrichtung oder an weiteren Elementen eine überhöhte Temperatur der Vorrichtung entstehen läßt.

Ausgehend von der Erkenntnis, daß die indirekt wirkenden Vorkehrungen zur Begrenzung der Temperatur im Meßwertaufnehmer und damit an der von ihm abgedeckten Körperstelle noch nicht voll befriedigen, ergab sich die der Erfindung zugrundeliegende Aufgabe, nämlich mit möglichst geringem technischen Aufwand eine Temperatursicherung mit höherer Zuverlässigkeit als bisher zu schaffen.

Diese Aufgabe ist erfindungsgemäß mit einem Meßwertaufnehmer der eingangs genannten Art gelöst, der die im kennzeichnenden Teil des Patentanspruchs 1 angegebenen Merkmale aufweist.

Dadurch, daß unmittelbar in den Meßwertaufnehmer ein elektronischer Schaltkreis eingebaut ist, der selbsttätig die Heizung unterbricht, wenn die Temperatur in seiner Umgebung einen vorgegebenen Wert überschreitet, ist gewährleistet, daß die vom Meßwertaufnehmer bedeckte Körperstelle nicht überhitzt und also nicht durch eine oberflächliche oder tiefergehende Verbrennung geschädigt wird. Diese Sicherheitsschaltung wirkt vor allem auch dann, wenn durch Defekte im Temperaturfühler oder im Heizleistungsregelkreis die Heizung außer Kontrolle geraten ist.

Der in den Meßwertaufnehmer eingebaute Schaltkreis unterbricht die Heizung, beispielsweise durch Unterbrechen oder Kurzschließen der Zuleitungen zum Heizelement, wenn ein bestimmter Schwellenwert der Temperatur überschritten ist.

Durch die unmittelbare Speisung des eine Überhitzung des Meßwertaufnehmers verhindernden elektronischen Schaltkreises aus der vorhandenen Stromversorgung für die elektrische Heizeinrichtung wird ein zusätzliches Leitungspaar im Verbindungskabel zwischen dem Meßgerät und dem Meßwertaufnehmer vermieden. Zwar könnte die Schaltungsanordnung bei direkter Speisung aus dem Meßgerät auch so getroffen sein, daß bei einem Bruch der dann notwendigen zusätzlichen Leitungsader in jedem Fall eine Abschaltung der Heizung erfolgt. Der Gesamtaufwand wäre jedoch größer und man würde von dem angestrebten Ziel weiter abrükken, nämlich die Anzahl der Leitungsadern im Verbindungskabel möglichst gering zu halten. Mit der Erfindung wird durch den unmittelbaren Einbau eines weiteren Temperaturfühlers und des zugeordneten elektronischen Schaltkreises eine unmittelbare Notabschaltung erreicht und damit die Betriebssicherheit auch ohne eine getrennte Stromversorgungsader im Verbindungskabel zwischen dem Meßgerät und dem Meßwertaufnehmer erreicht.

Durch die Stromversorgung für den elektronischen Schaltkreis aus der Heizstromversorgung des Meßwertaufnehmers wird einerseits mindestens eine zusätzliche Leitungsader, also auch eine nachteilige Verdickung des Verbindungskabels zwischen dem Meßgerät und dem Meßwertaufnehmer vermieden und das durch eine zusätzliche Leitungsader inherent vorhandene Sicherheitsproblem wird ebenfalls gelöst.

Bei der Direktspeisung des elektronischen Schaltkreises aus den Stromversorgungsleitungen für die Heizung wird eine Überheizung des Meßwertaufnehmers unmöglich, denn eine Unterbrechung der Stromversorgung für den elektronischen Schaltkreis bedeutet gleichzeitig eine Unterbrechung der Stromversorgung der Heizung.

Die Erfindung bietet also den Vorteil, daß eine zusätzliche sicherheitsvermindernde Zuleitung vermieden wird und es wird erreicht, daß bei einem Leiterbruch der elektronische Schaltkreis und die Heizung gleichzeitig unterbrochen werden, so daß ein alleiniger Ausfall des elektronischen Schaltkreises mit der Gefahr einer Überhitzung vermieden wird.

Bei einer vorteilhaften Ausgestaltungsform der Erfindung wird durch eine einfache ergänzende Schaltungsmaßnahme erreicht, daß die Stromzufuhr zur Heizung entweder ganz bis zu einer vom Gerätebenutzer einzuleitenden Maßnahme oder mindestens so lange unterbrochen wird, bis eine bestimmte obere Schwellenwerttemperatur, die an der Temperaturüberwachungseinheit eingestellt werden kann, wieder unterschritten ist.

Der von dem weiteren Temperaturfühler gesteuerte elektronische Schaltkreis läßt sich als integrierter Schaltkreis oder als Hybridschaltkreis auf kleinstem Raum im Gehäuse des Meßwertaufnehmers unterbringen.

Die Erfindung und vorteilhafte Einzelheiten werden nachfolgend unter Bezug auf die Zeichnung in beispielsweisen Ausführungsformen näher erläutert. Es zeigt

Fig. 1 in schematischer Schnittdarstellung ein Beispiel für einen Meßwertaufnehmer;

Fig. 2 das Prinzipschaltbild eines elektronischen Schaltkreises mit einem Temperaturfühler;

Fig. 3 eine etwas abgewandelte Ausführungsform des Schaltkreises nach Fig. 1 mit einer zusätzlichen Maßnahme zur bleibenden Abschaltung der Heizung im Meßwertaufnehmer nach Überschreiten eines bestimmten Schwellenwerts der Temperatur, und

Fig. 4 das Schaltbild der Fig. 2 mit einer beispielsweisen Einzeldarstellung des in Fig. 2 mit 50 bezeichneten Schwellenwertschalters.

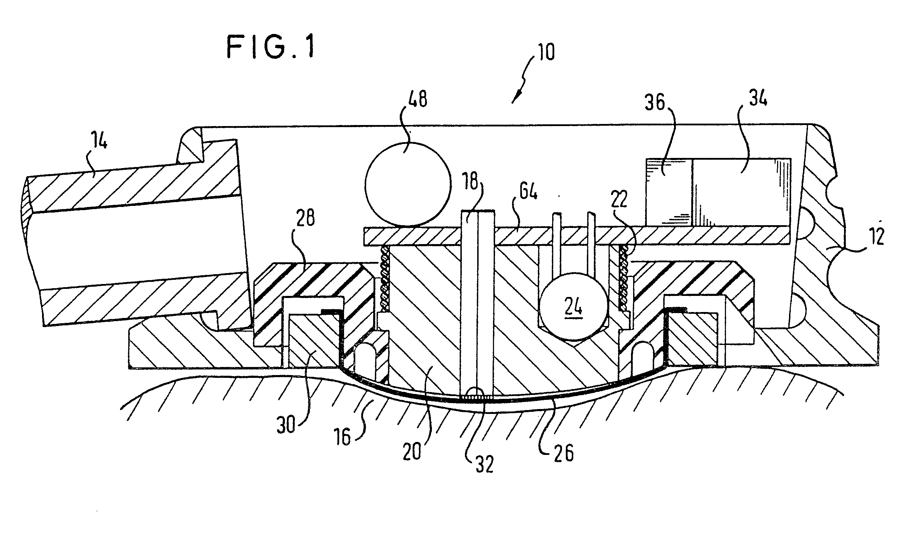

Die Fig. 1 zeigt in schematischer Schnittdarstellung einen physiologischen Meßwertaufnehmer 10, dessen einzelne Bauteile in einem Gehäuse 12 untergebracht sind. Der Meßwertaufnehmer ist üblicherweise an eine nicht gezeigte Überwachungseinheit über ein Kabel 14 mit einer Vielzahl von Adern verbunden, über welche einerseits die Stromversorgung der einzelnen Baugruppen sowie Meß- und Steuersignale übertragen werden. Der Meßwertaufnehmer 10 kann ein Polarometer zur transkutanen Bestimmung der Konzentration von Gasen, etwa des Partialdrucks von Sauerstoff im Blut eines Patienten sein. Ein typischer Vertreter eines solchen Sensors ist beispielsweise die sogenannte CLARK-Elektrode, die im wesentlichen die Form einer kreisrunden Platte von wenigen Millimetern Dicke und einigen Zentimetern Durchmesser aufweist. Während der Messung wird das die wesentlichen Komponenten des Aufnehmers enthaltende Gehäuse 12 in unmittelbaren Berührungskontakt mit der Haut 16 eines Patienten gebracht.

Im Gehäuse 12 des Meßwertaufnehmers 10 ist beispielsweise eine herkömmliche Trippelelektrode 18 vorzugsweise aus Platin eingebaut, die gegen einen Silberblock 20 isoliert ist. Ein als Wicklung 22 dargestelltes Heizelement umgibt den Silberblock 20. Diese und alle übrigen Bauelemente des Meßwertaufnehmers werden innerhalb des Gehäuses 12 durch Klammerelemente, durch Kleben oder in anderer bekannter Weise fixiert.

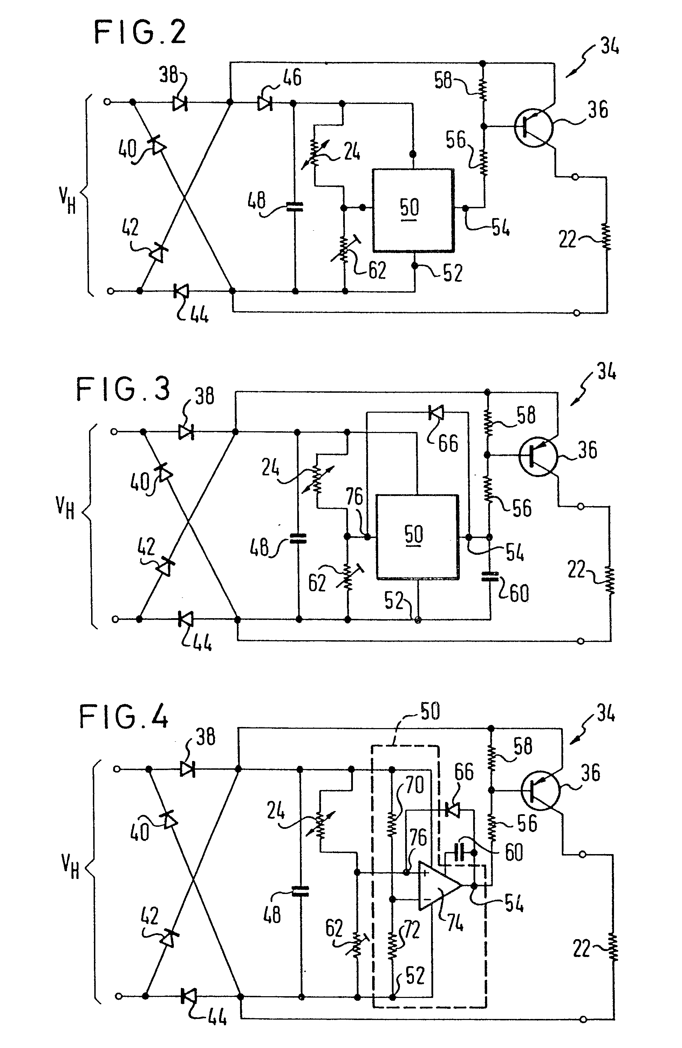

Die in der Zeichnung nicht dargestellten Einzelleiter im Kabel 14 übertragen unter anderem den polarometrischen Strom an ein geeignetes Aufzeichnungs- oder Anzeigeinstrument in der entfernt angeordneten nicht gezeigten Steuer- und Überwachungseinheit. Über andere Leiter im Kabel 14 erfolgt die Stromzufuhr zur Heizwicklung 22 über einen elektronischen Schalter 36, der über einen auf Temperaturänderungen ansprechenden Schaltkreis 34 gespeist wird, dessen Aufbau nachfolgend unter Bezug auf Fig. 2 in Einzelheiten erläutert wird.

Es sei betont, daß in der Darstellung der Fig. 1 einzelne Schaltkreiselemente lediglich schematisch, in den übrigen Figuren jedoch in Einzelheiten angegeben sind. Ein die Temperatur überwachendes Element, beispielsweise ein temperaturabhängiger Widerstand 24, etwa ein Thermistor, wirkt mit den übrigen noch zu erläuternden Baugruppen des Schaltkreises 34 und dem elektronischen Schalter 36 zusammen und dient zur Notabschaltung im Falle einer Überhitzung des Meßwertaufnehmers bzw. der von diesem bedeckten Hautfläche. Ein weiteres temperaturabhängiges Element (nicht gezeigt), beispielsweise ein temperaturabhängiger Widerstand vom gleichen Typ wie der Widerstand 24, ist ebenfalls innerhalb des Silberblocks angeordnet und über ein getrenntes Leiterpaar des Kabels 14 mit einem Thermostaten verbunden, um die Hauttemperatur innerhalb eines vorgegebenen Bereichs zu halten.

Die untere Oberfläche des Silberblocks 20 und der Trippelelektrode 18 sind durch eine dünne Membran 26 abgedeckt, die mittels eines durch einen Druckring 30 gesicherten Halterings 28 dicht abschließend gegen den Silberblock 20 vorgespannt ist. Im geringen Zwischenraum 32 zwischen der Membran 26 und der Trippelelektrode 18 ist ein geeigneter Elektrolyt eingebracht. Die Membran 26 kann aus Kunststoff beispielsweise aus Polyäthylenfolie, Polypropylenfolie oder Polytetrafluoräthylenfolie bestehen. Die wesentliche Eigenschaft der Membran 26 besteht darin, daß sie für die zu messenden Gase durchlässig, jedoch für den Elektrolyten undurchlässig ist.

Normalerweise wird die Temperatur des physiologischen Meßwertaufnehmers 10 in bekannter Weise durch den externen Thermostaten konstant gehalten. Der auf Temperaturänderungen ansprechende Schaltkreis 34, der innerhalb des Gehäuses 12 untergebracht ist und von dem temperaturabhängigen Widerstand 24 gesteuert wird, unterbricht die Stromversorgung zur Heizwicklung 22 oder schließt diese kurz wenn irgendein Fehlverhalten des Gerätes zu einem unerwarteten Temperaturanstieg führt. Dieser Schaltkreis wird nachfolgend in beispielsweisen Ausführungsformen und unter Bezug auf die Fig. 2 bis 4 beschrieben.

Der in den Fig. 2 bis 4 dargestellte von dem temperaturabhängigen Widerstand 24 gesteuerte elektronische Schaltkreis 34 besteht aus einem Schalttransistor 36, dessen Kollektor-Emitter-Strecke (Laststrecke) in Reihe zu einem Lastwiderstand 22 liegt, welcher die Heizwicklung des Meßwertaufnehmers darstellt oder diese enthält. Die Reihenschaltung aus Transistor 36 und Lastwiderstand 22 liegt zwischen dem Gleichspannungsausgang eines Vollweg-Brückengleichrichters, gebildet aus Dioden 38, 40, 42 und 44. Dieser Vollweg-Gleichrichter dient zur Gleichrichtung einer in der Regel impulsförmigen (getakteten) Wechselspannung $V_H$, welche der oben erwähnten unmittelbar aus der Stromversorgung für die Heizwicklung abgeleiteten Hilfsspannung entspricht. Sofern das Vorzeichen von $V_H$ positiv ist, kann der Brückengleichrichter 38 bis 44 entfallen. In der Schaltung nach Fig. 2 dient die Reihenschaltung einer Diode 46 und eines Kondensators 48 zwischen den Gleichspannungsausgängen des Vollweg-Gleichrichters 38 bis 44 zur Glättung der Betriebsspannung für einen Schwellenwertschalter, z. B. Schmitt-Trigger 50, dessen Ausgangskreis zwischen den Anschlüssen 52, 54 in Reihe zu Widerständen 56, 58 liegt. Die Basis des Transistors 36 ist am Verbindungspunkt zwischen den Widerständen 56 und 58 angeschlossen. Ersichtlicherweise ist der jeweilige Arbeitspunkt des Transistors 36 bestimmt durch das jeweilige Widerstandsverhältnis $R_{58} : (R_{56} + R_{52 \to 54})$, wobei mit $R_{52 \to 54}$ der Widerstand zwischen den mit Bezugshinweis 52 bzw. 54 angegebenen Anschlüssen des Schwellenwertschalters 50 bezeichnet ist. Ein in den Fig. 3 und 4 im Ausgangskreis des Schwellenwertschalters 50 liegender Kondensator 60 dient als Störschutz und zur Ansprechverzögerung beim Einschalten der Versorgungsspannung.

Der Schalt- oder Einsatzpunkt des Schwellenwertschalters 50 wird durch das Teilerverhältnis von zwei zwischen der gleichgerichteten Hilfsspannung in Reihe liegenden Widerständen 24 und 62 bestimmt, von denen der Widerstand 24 der bereits erwähnte temperaturabhängige, möglichst nahe der Meßoberfläche des Meßwertaufnehmers angeordnete Widerstand, also beispielsweise ein Thermistor ist, während am Einstellwiderstand 62 die Schaltschwelle des Schwellenwertschalters 50 eingestellt werden kann. Bei der Ausführungsform nach Fig. 3 ist zwischen dem Ausgang 54 und dem Steuereingang 76 des Schwellenwertschalters 50 eine Diode 66 geschaltet, welche nach Überschreiten der einstellbaren Schaltschwelle für eine bleibende Abschaltung sorgt, bis durch den Gerätebenutzer die Ursache einer möglichen Übererwärmung des Meßwertaufnehmers beseitigt ist. Durch die Diode 66 wird mittels einer starken positiven Rückkoppelung ein Thyristoreffekt erzielt.

Die Fig. 4 zeigt ein konkretes Ausführungsbeispiel für den Schwellenwertschalter 50. Wie dargestellt, kann die Reihenschaltung der Widerstände 24 und 62 durch parallel geschaltete Reihenwiderstände 70 und 72 zu einer Widerstandsbrücke ergänzt sein, in deren Querzweig die beiden Eingänge eines Differenzverstärkers 74 liegen. Anstelle eines Differenzverstärkers kann auch ein Komparator verwendet werden.

Die Schaltung nach Fig. 4 arbeitet wie folgt: Überschreitet die Temperatur des Widerstands 24 und damit die Spannung an Punkt 76 den durch $R_{70}/R_{72}$ vorgegebenen Schwellenwert, so wird der Ausgang 54 von 74 positiv, sperrt also den Transistor 36 und unterbricht damit den Heizstrom durch den Lastwiderstand 22. Gleichzeitig wird über die Diode 66 der nicht-invertierende Eingang des Differenzverstärkers 74 durch positive Rückkoppelung auf positiveres Potential gebracht, was ein sauberes Schaltverhalten bewirkt, und durch die Klemmwirkung von der Diode 66 ein Absinken des Potentials am Punkt 76 auch dann verhindert, wenn die Temperatur sinkt und der Widerstand 24 hochohmiger wird. Der Kondensator 60 wirkt hier als Integrator mit der Ausgangsstufe des Komparators oder Differenzverstärkers 74.

## Patentansprüche

1. Meßwertaufnehmer für physiologische Meßgrößen mit einer elektrischen Heizeinrichtung in dem Meßwertaufnehmer und einem in dem Meßwertaufnehmer angeordneten Temperaturfühler, der mit einer außerhalb des Meßwertaufnehmers angeordneten Temperaturregeleinrichtung über Meßleitungen verbunden ist, dadurch gekennzeichnet, daß in dem Meßwertaufnehmer ein weiterer Temperaturfühler (24) sowie ein von diesem steuerbarer elektronischer Schaltkreis (34) vorgesehen sind zur Notabschaltung der Heizeinrichtung (22) bei Überhitzung, wobei die zum Betrieb des elektronischen Schaltkreises benötigte elektrische Hilfsenergie aus der vorhandenen Stromversorgung für die elektrische Heizeinrichtung abgeleitet ist.

2. Meßwertaufnehmer nach Anspruch 1, gekennzeichnet durch eine Schaltungsgruppe (50, 66) in dem elektronischen Schaltkreis, welche die Unterbrechung der Stromzufuhr zur Heizung vom Erreichen der Schwellentemperatur an bis zum Eingreifen durch den Benutzer dauernd sicherstellt (Thyristoreffekt).

3. Meßwertaufnehmer nach Anspruch 1, gekennzeichnet durch eine Schaltungseinheit in dem elektronischen Schaltkreis, welche die Stromzufuhr zur Heizung vom Erreichen der Schwellenwerttemperatur an so lange unterbricht, bis die Schwellenwerttemperatur wieder unterschritten wird.

4. Meßwertaufnehmer nach Anspruch 2, dadurch gekennzeichnet, daß die Schaltungsgruppe einen Schwellenwertschalter (50) enthält, durch welchen die Heizung vom Erreichen der Schwellenwerttemperatur an so lange außer Funktion gesetzt wird, bis eine um den Betrag der Schalthysterese verminderte Temperatur wieder erreicht wird.

5. Meßwertaufnehmer nach Anspruch 4, dadurch gekennzeichnet, daß der Eingang des Schwellenwertschalters (50) im Querzweig einer von der Heizstromversorgung $(V_H)$ gespeisten Widerstandsmeßbrücke (24, 62, 70, 72) liegt, deren einer Zweig einen temperaturabhängigen Widerstand (24) enthält.

6. Meßwertaufnehemer nach Anspruch 4 oder 5, dadurch gekennzeichnet, daß der elektronische Schaltkreis einen Transistor (36) enthält, dessen Kollektor-Emitterstrecke in Reihe zu einer Heizwicklung (22) im Meßwertaufnehmer geschaltet ist und in dessen Steuerkreis der Schwellenwertschalter (50) liegt.

7. Meßwertaufnehmer nach einem der Ansprüche 4 bis 6, dadurch gekennzeichnet, daß der Schwellenwertschalter ein Differenzverstärker ist.

8. Meßwertaufnehmer nach Anspruch 7, dadurch gekennzeichnet, daß zur Festhaltung der Abschaltung nach Überschreiten des Temperaturschwellenwerts der Ausgang des Differenzverstärkers (74) über eine Diode (66) mit dem nichtinvertierenden Eingang des Differenzverstärkers (74) verbunden ist.

9. Meßwertaufnehmer nach einem der Ansprüche 4 bis 8, dadurch gekennzeichnet, daß im Ausgangskreis des Schwellwertschalters (50) ein Kondensator (60) vorgesehen ist, der als Störschutz und zur Ansprechverzögerung beim Einschalten der Versorgungsspannung $(V_H)$ dient.

10. Meßwertaufnehmer nach Anspruch 2 oder 4, dadurch gekennzeichnet, daß ein Glättungsglied (46, 48) eine Versorgung des elektronischen Schaltkreises aus einer getakteten Versorgungsspannung ermöglicht.

**Claims**

1. A measurement sensor for physiological parameters, having an electric heating device in the measurement sensor and a temperature sensor which is arranged in the measurement sensor and which is connected via measuring lines to a temperature control device arranged outside the measurement sensor, characterised in that in the measurement sensor another temperature sensor (24) and an electronic circuit (34), which can be controlled by this temperature sensor, are provided for switching off, in an emergency, the heating device (22) in the case of overheating, the auxiliary electric energy required for operating the electronic circuit being derived from the existing power supply for the electric heating device.

2. A measurement sensor according to claim 1, characterised by a circuit group (50, 66) in the electronic circuit, which circuit group permanently ensures the interruption of the power supply to the heating device from the time the threshold temperature has been reached until the user intervenes (thyristor effect).

3. A measurement sensor according to claim 1, characterised by a circuit unit in the electronic circuit, which circuit unit interrupts the power supply to the heating device from the time the threshold temperature has been reached until the temperature again drops below the threshold temperature.

4. A measurement sensor according to claim 2, characterised in that the circuit group contains a threshold switch (50) by means of which the heating device is made inoperative from the time the threshold temperature has been reached until a temperature which is reduced by the amount of switching hysteresis, is reached again.

5. A measurement sensor according to claim 4, characterised in that the input of the threshold switch (50) is located in the shunt branch of a resistance measuring bridge (24, 62, 70, 72) which is fed from the heating power supply $(V_H)$ and one branch of which contains a temperature-dependent resistance (24).

6. A measurement sensor according to claim 4 or 5, characterised in that the electronic circuit contains a transistor (36), the collector-emitter path of which is connected in series with a heater winding (22) in the measurement sensor and in the control circuit of which the threshold switch (50) is located.

7. A measurement sensor according to one of claims 4 to 6, characterised in that the threshold switch is a differential amplifier.

8. A measurement sensor according to claim 7, characterised in that for holding the switched-off condition after the temperature threshold has been exceeded the output of the differential amplifier (74) is connected via a diode (66) to the non-inverting input of the differential amplifier (74).

9. A measurement sensor according to one of claims 4 to 8, characterised in that in the output circuit of the threshold switch (50) a capacitor (60) is provided which is used as interference protection and for delaying the response when the supply voltage ($V_H$) is switched on.

10. A measurement sensor according to claim 2 or 4, characterised in that a smoothing element (46, 48) makes it possible to supply the electronic circuit from a clocked supply voltage.


## Revendications

1. Capteur de mesure pour mesures physiologiques comportant un dispositif de chauffage électrique incorporé au capteur et, disposée dans le capteur, une sonde de température qui est reliée par câbles à un dispositif de réglage de la température installée en dehors du capteur, caractérisé par le fait qu'une autre sonde de température (24) est prévue dans le capteur ainsi qu'un circuit électronique (34) asservi à cette sonde pour couper, en cas de surchauffe, l'alimentation électrique du dispositif de chauffage (22), l'énergie électrique auxiliaire nécessaire à l'alimentation du circuit électronique provenant de l'alimentation électrique du dispositif de chauffage.

2. Capteur de mesure selon la revendication 1, caractéerisé par le fait qu'un groupe de coupure (50, 60) dans le circuit électronique assure de façon durable l'interruption de l'alimentation en courant de chauffage dès que la température de seuil est atteinte jusqu'à l'intervention de l'utilisateur (effet de thyristor).

3. Capteur de mesure selon la revendication 1, caractérisé par le fait qu'une unité de coupure dans le circuit électronique interrompt l'alimentation en courant de chauffage dès que la température de seuil est atteinte et aussi longtemps que la température ne revient en-dessous de la valeur de seuil.

4. Capteur de mesure selon la revendication 2, caractérisé par le fait que le groupe de coupure contient un interrupteur à valeur de seuil (50) qui met hors service le chauffage dès que la température de seuil est atteinte et jusqu'à ce que soit de nouveau atteinte une température diminuée de l'hystérésis de coupure.

5. Capteur de mesure selon la revendication 4, caractérisé par le fait que l'entrée de l'interrupteur à valeur de seuil (50) se trouve dans une branche en dérivation d'un pont de résistances (24, 62, 70, 72), alimenté par le courant de chauffage ($V_H$), et dont une branche contient une résistance (24) variable en fonction de la température.

6. Capteur de mesure selon la revendication 4 ou 5, caractérisé par le fait que le circuit électronique contient un transistor (36) dont le trajet collecteur-émetteur est branché en série avec une bobine de chauffage (22) dans le capteur et dans le circuit de commande duquel se trouve placé l'interrupteur à valeur de seuil (50).

7. Capteur de mesure selon l'une des revendications 4 à 6, caractérisé par le fait que l'interrupteur à valeur de seuil est un amplificateur différentiel.

8. Capteur de mesure selon la revendication 7, caractérisé par le fait que pour maintenir la coupure lorsque la valeur de seuil de température a été franchie, la sortie de l'amplificateur différentiel (74) est reliée par une diode (66) à l'entrée de non-inversion de l'amplificateur différentiel (74).

9. Capteur de mesure selon l'une des revendications 4 à 8, caractérisé par le fait que dans le circuit de sortie de l'interrupteur à valeur de seuil (50), est prévu un condensateur (60) qui sert d'anti-parasitage et de retardateur d'amorçage lors de l'établissement de la tension d'alimentation ($V_H$).

10. Capteur de mesure selon la revendication 2 ou 4, caractérisé par le fait qu'un élément de lissage (46, 48) permet une alimentation du circuit électronique à partir d'une tension d'alimentation intermittente.

FIG.1

FIG.2

FIG.3

FIG.4